# EUROPEAN PATENT APPLICATION

(11) **EP 4 364 695 A1**
(43) Date of publication of application: **08.05.2024**
(21) Application number: 23207716.4
(22) Date of filing: 03.11.2023
(51) Int. Cl.: A61F 2/24

(54) **VALVE REPAIR CLIP WITH LEAFLET CAPTURE CONFIRMATION**

(30) Priority: 03.11.2022 US 202263382206 P
(71) Applicant: Evalve Inc., Santa Clara, California 95054 (US)
(72) Inventor: DATTA, Saurabh, PLEASANTON, CA 94566 (US); SNELL, Anna Maria, BELMONT, CA 94002 (US); ABUNASSAR, Chad J., ALAMEDA, CA 94501 (US)
(74) Representative: Boult Wade Tennant LLP

(57) **Abstract**

A heart valve repair clip includes a stud, two arms operatively coupled to the stud and capable of transitioning between an open condition and a closed condition, and includes an indicator coupled to each of the two arms and moveable relative thereto.

## Description

### CROSS-REFERENCE TO RELATED APPLICATIONS

The present application claims priority to U.S. Provisional Serial No. 63/382,206, filed November 3, 2022, the disclosure of which is hereby incorporated by reference in its entirety as if fully set forth herein.

### BACKGROUND

Mitral valve regurgitation may be characterized by retrograde flow from the left ventricle of a heart through a compromised mitral valve into the left atrium. During a normal cycle of heart contraction (systole), the mitral valve ideally acts as a one-way valve to prevent flow of oxygenated blood back into the left atrium. In this way, the oxygenated blood is pumped into the aorta through the aortic valve. Valve regurgitation may significantly decrease the pumping efficiency of the heart, placing the patient at risk of severe, progressive heart failure.

Mitral valve regurgitation can result from a number of different mechanical defects in the mitral valve or the left ventricular wall. The valve leaflets, the valve chordae which connect the leaflets to the papillary muscles, the papillary muscles or the left ventricular wall may be damaged or otherwise dysfunctional. Commonly, the valve annulus may be damaged, dilated, or weakened limiting the ability of the mitral valve to close adequately against the high pressures of the left ventricle.

Common treatments for mitral valve regurgitation rely on valve replacement or repair including leaflet and annulus remodeling, the latter generally referred to as valve annuloplasty. Another technique for mitral valve repair which relies on suturing adjacent segments of the opposed valve leaflets together is referred to as the "bow-tie" or "edge-to-edge" technique. While all these techniques can be very effective, they usually rely on open heart surgery where the patient's chest is opened, typically via a sternotomy, and the patient placed on cardiopulmonary bypass. The need to both open the chest and place the patient on bypass is traumatic and has associated high mortality and morbidity.

Alternatively, mitral valve regurgitation may be corrected by transcatheter delivery of an implant that facilitates full closure of the mitral valve during each heart contraction cycle. Transcatheter delivery can be a complicated process requiring close attention and many inputs and manipulations from an implanter, interventionalist, or physician, which will collectively be referred to with the term "physician" in the remainder of this disclosure. Some physicians may therefore find utility in a configuration of the implant and delivery system that allows for proper confirmation of leaflet placement and capture.

Specifically, proper training is desirable for adequate leaflet grasping during certain repair procedures such as edge-to-edge repair procedures. Typically, an operator will actively monitor leaflet insertion into the proper position adjacent the proximal and/or distal arms, for example, through an ultrasound-based imaging method. This process may be time-consuming and difficult due to the presence of artifact-causing metallic components of the device right next to leaflet tissue. Due to difficulty of imaging highly mobile leaflets and artifacts it is difficult to ensure adequate leaflet insertion and grasp. This leads to higher probability of single leaflet device attachment (SLDA) or similar complications.

### BRIEF SUMMARY

In some examples, a valve clip includes a stud, two arms operatively coupled to the stud and capable of transitioning between an open condition and a closed condition, and at least one sensor coupled to each of the two arms.

In some examples, a valve clip includes a stud, two arms operatively coupled to the stud and capable of transitioning between an open condition and a closed condition, and an indicator coupled to each of the two arms and moveable relative thereto.

### BRIEF DESCRIPTION OF THE DRAWINGS

FIG. 1 illustrates the left ventricle and left atrium of the heart during systole.
FIG. 2A illustrates free edges of leaflets in normal coaptation, and FIG. 2B illustrates the free edges in regurgitative coaptation.
FIG. 3A-3C illustrate grasping of the leaflets with a fixation device, inversion of the distal elements of the fixation device and removal of the fixation device, respectively.
FIG. 4 illustrates the position of the fixation device in a desired orientation relative to the leaflets.
FIGS. 5, 6A-B and 7 illustrate an embodiment of a fixation device in various positions.
FIGS. 8A-8B illustrate an embodiment of the fixation device wherein some or all of the components are molded as one part.
FIG. 9 illustrates another embodiment of the fixation device of the present disclosure.
FIGS. 10A-10B, 11A-11B, 12A-12B, 13A-13B, 14-16 illustrate embodiments of a fixation device in various possible positions during introduction and placement of the device within the body to perform a therapeutic procedure.
FIGS. 17A-17C illustrate a covering on the fixation device wherein the device is in various positions.
FIG. 18 illustrates another embodiment of the fixation device of the present disclosure having an electromechanical sensor.
FIG. 19A-D illustrate the use of a fixation device having a sensor.
FIG. 20A-B illustrate another embodiment of a fixation device having a mechanical indicator.
FIG. 21A-D illustrate the position of the indicator of FIGS. 20-B in various condition.
FIG. 22A-B illustrate an inner view of a mechanical indicator without tissue, and being deflected due to inserted tissue, respectively.

### DETAILED DESCRIPTION

When used in connection with a delivery device for transporting a device into a patient, the terms "proximal" and "distal" are to be taken as relative to the user of the delivery devices. "Proximal" is to be understood as relatively close to the user, and "distal" is to be understood as relatively farther away from the user. When used in connection with a fixation device, the terms "proximal" and "distal" are to be taken as relative to the site of treatment. "Proximal" is to be understood as relatively close to the treatment site, and "distal" is to be understood as relatively farther away from the treatment site. As used herein, the terms "substantially," "generally," "approximately," and "about" are intended to mean that slight deviations from absolute are included within the scope of the term so modified. Throughout the disclosure, the mitral valve is described in an illustrative manner. Clips may be similarly used to treat the tricuspid valve to reduce regurgitation in the right side of the heart. This tricuspid valve repair approach is particularly hindered by poor imaging due to the unfavorable anatomy of the heart in relation to the esophagus. A trans-esophageal echocardiography probe can be pressed favorably toward the left side of the heart to obtain adequate imaging of the mitral valve. This is not the case for the tricuspid valve, so imaging is generally poorer. For this reason, a sensor may provide a special benefit for users to gain confidence in implanting clips in tricuspid repair procedures. Thus, the disclosure is not limited to mitral valve clips, but similar techniques may also be used to ensure proper attachment of other clips, valves or other devices in cardiac and other medical applications.

### I. Cardiac Physiology

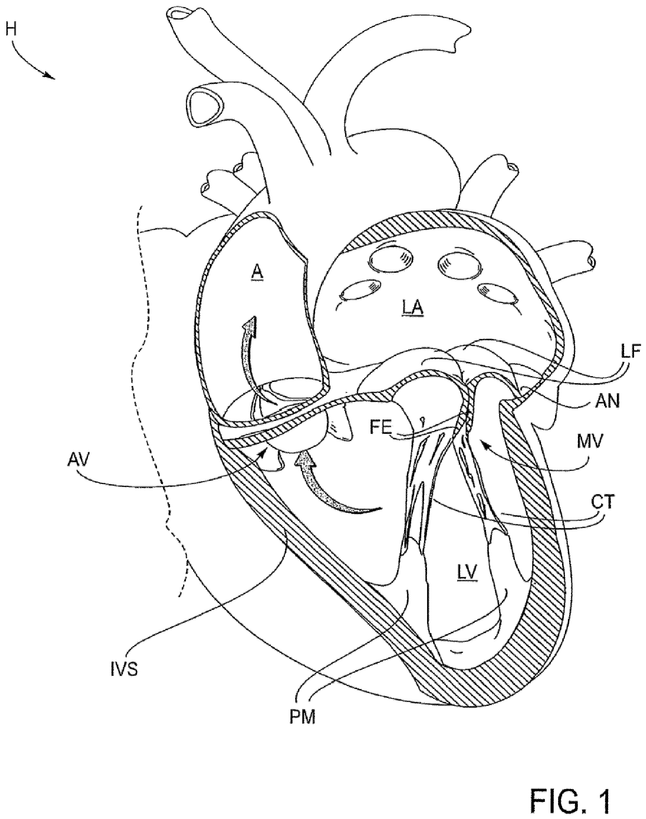

The left ventricle LV of a normal heart H in systole is illustrated in FIG. 1. The left ventricle LV is contracting and blood flows outwardly through the aortic valve AV in the direction of the arrows. Back flow of blood or "regurgitation" through the mitral valve MV is prevented since the mitral valve is configured as a "check valve" which prevents back flow when pressure in the left ventricle is higher than that in the left atrium LA. The mitral valve MV comprises a pair of leaflets having free edges FE which meet evenly to close, as illustrated in FIG. 1. The opposite ends of the leaflets LF are attached to the surrounding heart structure along an annular region referred to as the annulus AN. The free edges FE of the leaflets LF are secured to the lower portions of the left ventricle LV through chordae tendinae CT (referred to hereinafter as the chordae) which include plurality of branching tendons secured over the lower surfaces of each of the valve leaflets LF. The chordae CT in turn, are attached to the papillary muscles PM which extend upwardly from the lower portions of the left ventricle and intraventricular septum IVS.

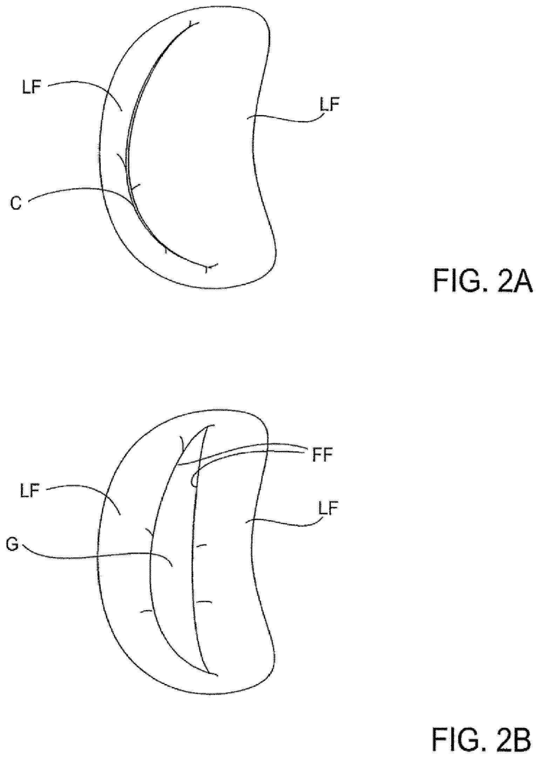

A number of structural defects in the heart can cause mitral valve regurgitation. Regurgitation occurs when the valve leaflets do not close properly allowing leakage from the ventricle into the atrium. As shown in FIG. 2A, the free edges of the anterior and posterior leaflets normally meet along a line of coaptation C. An example of a defect causing regurgitation is shown in FIG. 2B. Here an enlargement of the heart causes the mitral annulus to become enlarged, making it impossible for the free edges FE to meet during systole. This results in a gap G which allows blood to leak through the valve during ventricular systole. Ruptured or elongated chordae can also cause a valve leaflet to prolapse since inadequate tension is transmitted to the leaflet via the chordae. While the other leaflet maintains a normal profile, the two valve leaflets do not properly meet and leakage from the left ventricle into the left atrium will occur. Such regurgitation can also occur in patients who have suffered ischemic heart disease where the left ventricle does not contract sufficiently to effect proper closure.

### II. General Overview

The present disclosure provides methods and devices for grasping, approximating and fixating tissues such as valve leaflets to treat cardiac valve regurgitation, particularly mitral valve regurgitation. The present disclosure also provides features that allow repositioning and removal of the device if so desired, particularly in areas where removal may be hindered by anatomical features such as chordae CT. Such removal would allow the surgeon to reapproach the valve in a new manner if so desired.

Grasping will preferably be atraumatic providing a number of benefits. By atraumatic, it is meant that the devices and methods of the disclosure may be applied to the valve leaflets and then removed without causing any significant clinical impairment of leaflet structure or function. The leaflets and valve continue to function substantially the same as before the disclosure was applied. Thus, some minor penetration or denting of the leaflets may occur using the disclosure while still meeting the definition of "atraumatic". This enables the devices of the disclosure to be applied to a diseased valve and, if desired, removed or repositioned without having negatively affected valve function. In addition, it will be understood that in some cases it may be necessary or desirable to pierce or otherwise permanently affect the leaflets during either grasping, fixing or both. In some of these cases, grasping and fixation may be accomplished by a single device. Although a number of embodiments are provided to achieve these results, a general overview of the basic features will be presented herein. Such features are not intended to limit the scope of the disclosure and are presented with the aim of providing a basis for descriptions of individual embodiments presented later in the application.

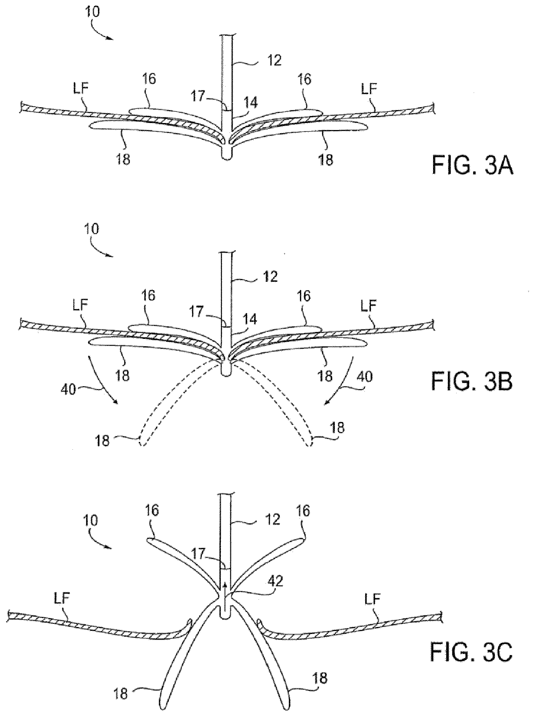

The devices and methods of the disclosure rely upon the use of an interventional tool that is positioned near a desired treatment site and used to grasp the target tissue. In endovascular applications, the interventional tool is typically an interventional catheter. In surgical applications, the interventional tool is typically an interventional instrument. In preferred embodiments, fixation of the grasped tissue is accomplished by maintaining grasping with a portion of the interventional tool which is left behind as an implant. While the disclosure may have a variety of applications for tissue approximation and fixation throughout the body, it is particularly well adapted for the repair of valves, especially cardiac valves such as the mitral valve. Referring to FIG. 3A, an interventional tool 10, having a delivery device, such as a shaft 12, and a fixation device 14, is illustrated having approached the mitral valve MV from the atrial side and grasped the leaflets LF. The mitral valve may be accessed either surgically or by using endovascular techniques, and either by a retrograde approach through the ventricle or by an antegrade approach through the atrium, as described above. For illustration purposes, an antegrade approach is described.

The fixation device 14 is releasably attached to the shaft 12 of the interventional tool 10 at its distal end. When describing the devices of the disclosure herein, "proximal" shall mean the direction toward the end of the device to be manipulated by the user outside the patient's body, and "distal" shall mean the direction toward the working end of the device that is positioned at the treatment site and away from the user. With respect to the mitral valve, proximal shall refer to the atrial or upstream side of the valve leaflets and distal shall refer to the ventricular or downstream side of the valve leaflets.

The fixation device 14 typically comprises proximal elements 16 (or gripping elements) and distal elements 18 (or fixation elements) which protrude radially outward and are positionable on opposite sides of the leaflets LF as shown so as to capture or retain the leaflets therebetween. The proximal elements 16 are preferably comprised of cobalt chromium, nitinol or stainless steel, and the distal elements 18 are preferably comprised of cobalt chromium or stainless steel, however any suitable materials may be used. The fixation device 14 is coupleable to the shaft 12 by a coupling mechanism 17. The coupling mechanism 17 allows the fixation device 14 to detach and be left behind as an implant to hold the leaflets together in the coapted position.

In some situations, it may be desired to reposition or remove the fixation device 14 after the proximal elements 16, distal elements 18, or both have been deployed to capture the leaflets LF. Such repositioning or removal may be desired for a variety of reasons, such as to reapproach the valve in an attempt to achieve better valve function, more optimal positioning of the device 14 on the leaflets, better purchase on the leaflets, to detangle the device 14 from surrounding tissue such as chordae, to exchange the device 14 with one having a different design, or to abort the fixation procedure, to name a few. To facilitate repositioning or removal of the fixation device 14 the distal elements 18 are releasable and optionally invertible to a configuration suitable for withdrawal of the device 14 from the valve without tangling or interfering with or damaging the chordae, leaflets or other tissue. FIG. 3B illustrates inversion wherein the distal elements 18 are movable in the direction of arrows 40 to an inverted position. Likewise, the proximal elements 16 may be raised, if desired. In the inverted position, the device 14 may be repositioned to a desired orientation wherein the distal elements may then be reverted to a grasping position against the leaflets as in FIG. 3A. Alternatively, the fixation device 14 may be withdrawn (indicated by arrow 42) from the leaflets as shown in FIG. 3C. Such inversion reduces trauma to the leaflets and minimizes any entanglement of the device with surrounding tissues. Once the device 14 has been withdrawn through the valve leaflets, the proximal and distal elements may be moved to a closed position or configuration suitable for removal from the body or for reinsertion through the mitral valve.

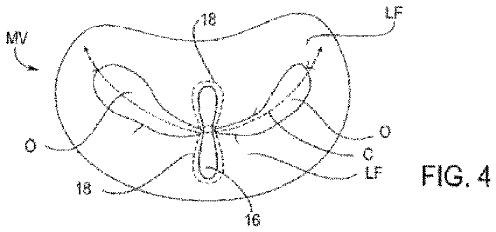

FIG. 4 illustrates the position of the fixation device 14 in a desired orientation in relation to the leaflets LF. This is a short-axis view of the mitral valve MV from the atrial side, therefore, the proximal elements 16 are shown in solid line and the distal elements 18 are shown in dashed line. The proximal and distal elements 16, 18 are positioned to be substantially perpendicular to the line of coaptation C. The device 14 may be moved roughly along the line of coaptation to the location of regurgitation. The leaflets LF are held in place so that during diastole, as shown in FIG. 4, the leaflets LF remain in position between the elements 16, 18 surrounded by openings O which result from the diastolic pressure gradient. Advantageously, leaflets LF are coapted such that their proximal or upstream surfaces are facing each other in a vertical orientation, parallel to the direction of blood flow through mitral valve MV. The upstream surfaces may be brought together so as to be in contact with one another or may be held slightly apart, but will preferably be maintained in the vertical orientation in which the upstream surfaces face each other at the point of coaptation. This simulates the double orifice geometry of a standard surgical bow-tie repair. Color Doppler echo will show if the regurgitation of the valve has been reduced. If the resulting mitral flow pattern is satisfactory, the leaflets may be fixed together in this orientation. If the resulting color Doppler image shows insufficient improvement in mitral regurgitation, the interventional tool 10 may be repositioned. This may be repeated until an optimal result is produced wherein the leaflets LF are held in place.

Once the leaflets are coapted in the desired arrangement, the fixation device 14 is then detached from the shaft 12 and left behind as an implant to hold the leaflets together in the coapted position. As mentioned previously, the fixation device 14 is coupled to the shaft 12 by a coupling mechanism 17. Other coupling mechanisms are described in U.S. Patent No. 9,510,829, which is hereby incorporated by reference in its entirety as if fully set forth herein.

### III. Fixation Device

### A. Introduction and Placement of Fixation Device

The fixation device 14 is delivered to the valve or the desired tissues with the use of a delivery device. The delivery device may be rigid or flexible depending on the application. For endovascular applications, the delivery device comprises a flexible delivery catheter which will be described in later sections. Typically, however, such a catheter comprises a shaft, having a proximal end and a distal end, and a fixation device releasably attached to its distal end. The shaft is usually elongate and flexible, suitable for intravascular introduction. Alternatively, the delivery device may comprise a shorter and less flexible interventional instrument which may be used for trans-thoracic surgical introduction through the wall of the heart, although some flexibility and a minimal profile will generally be desirable. A fixation device is releasably coupleable with the delivery device as illustrated in FIG. 3A. The fixation device may have a variety of forms, a few embodiments of which will be described herein.

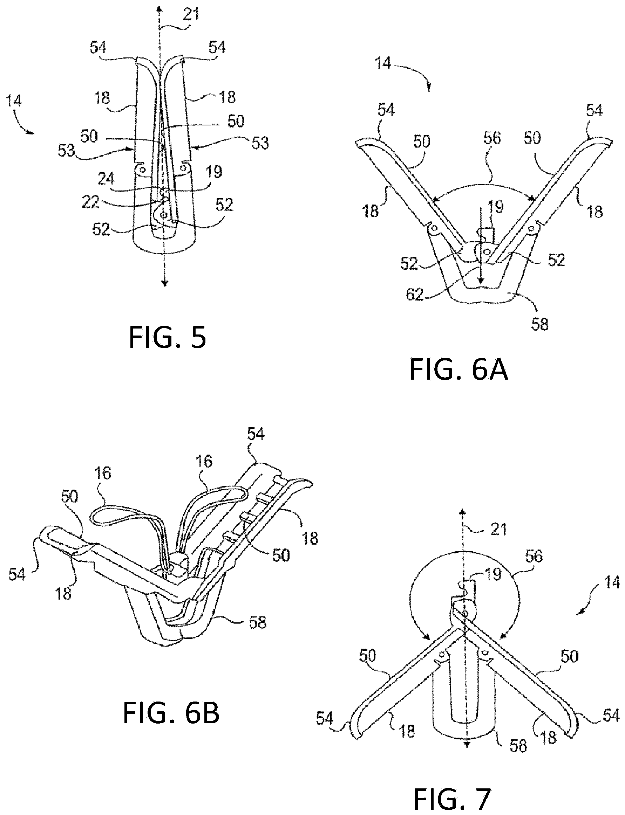

FIGS. 5, 6A-B and 7 illustrate an embodiment of a fixation device 14 in various positions or configurations. FIG. 5 illustrates the fixation device 14 in a closed configuration for delivery through the patient's vasculature and, in this example, through the mitral valve. The fixation device 14 includes a coupling member 19 which allows detachment of the fixation device 14 for implantation. In this example, the coupling member 19 is shown to include the lower shaft 22 and mating surface 24, and therefore the coupling member 19 would function similarly as described above. The fixation device 14 also includes a pair of opposed distal elements 18, each distal element 18 having an engagement surface 50 facing inwardly toward the opposed distal element 18 in the closed configuration. Distal elements 18 preferably comprise elongate arms 53, each arm having a proximal end 52 rotatably connected to the coupling member 19 and a free end 54. Suitable connections for arms 53 to coupling member 19 include pins, living hinges, or other known rotational connection mechanisms. In the closed configuration of FIG. 5, free ends 54 point in a first direction such that the arms 53 and engagement surfaces 50 are nearly parallel to each other and to an axis 21, and preferably are angled slightly inwardly toward each other. In a preferred embodiment, when tissue is not present between arms 53, the arms 53 may be closed until free ends 54 either touch each other or engage shaft 12 when fixation device 14 is attached thereto, thereby minimizing the profile of the fixation device 14 for passage through a delivery device.

FIGS. 6A-B illustrate the fixation device 14 in an open position wherein the engagement surfaces 50 are disposed at a separation angle 56 apart, wherein the separation angle 56 is typically up to approximately 180 degrees, preferably up to 90-180 degrees, and arms 53 are disposed generally symmetrically relative to axis 21. The arms 53 may be movable to the open position by a variety of actuation mechanisms. For example, a plunger or actuator rod may be advanced through the coupling member 19, as indicated by arrow 62, so as to engage a spring or spring loaded actuation mechanism 58 which is attached to the distal elements 18. By exerting a force against the actuation mechanism 58, the distal elements 18 are rotated relative to coupling member 19. The distal elements 18 may be held in this open position by the actuator rod against the resistance provided by the spring of the actuation mechanism 58 which biases the distal elements 18 toward the closed position of FIG. 5 when the distal elements 18 are less than 180 degrees apart. The spring loading of the actuation mechanism 58 resists outward movement of the actuation mechanism 58 and urges the device 14 towards the closed position.

In this embodiment, proximal elements 16 comprise resilient loop-shaped wire forms biased outwardly and attached to the coupling member 19 so as to be biased to an open position shown in FIG. 6B but movable rotationally inwardly when arms 53 are closed. The wire forms may be flexible enough to be rigidly attached to coupling member 19 and resiliently deflectable inwardly, or they may be attached by a rotational coupling such as a pin or living hinge. In use, leaflets LF are positioned between the proximal elements 16 and distal elements 18. Once, the leaflets LF are positioned between the proximal and distal elements 16, 18, the distal elements 18 may be closed, compressing the leaflets between engagement surfaces 50 and proximal elements 18. Depending upon the thickness of the leaflets, the arrangements of the leaflets, the position of the fixation device on the leaflets and other factors, the arms 53 may be maintained in the open position of FIGS. 6A-B, moved to the fully closed position of FIG. 5, or placed in any of various positions in between so as to coapt the leaflets LF and hold them in the desired position with the desired degree of force. In any case, the fixation device 14 will remain in place as an implant following detachment from the delivery catheter.

In some situations, as previously mentioned, it may be desirable to reopen the fixation device 14 following initial placement. To reopen the device 14, the actuator rod may be readvanced or reinserted through the coupling member 19 and readvanced to press against the actuation mechanism 58, as previously indicated by arrow 62 in FIG. 6A. Again, such advancement applies a force against the actuation mechanism 58 in the manner described above thus moving arms 53 outwardly to release force against leaflets and move engagement surfaces 50 away from proximal elements 16. The leaflets are then free to move relative to fixation device 14. The fixation device 14 may then be repositioned as desired and the actuator rod refracted to reclose the distal elements 18 to coapt the leaflets.

Under some circumstances, it may be further desirable to withdraw the fixation device 14 back through the valve or completely from the patient following initial insertion through the valve. Should this be attempted with the clip in the closed or open positions illustrated in FIGS. 5, 6A-B and 7, there may be a risk that arms 53 could interfere or become entangled with the chordae, leaflets or other tissues. To avoid this, the fixation element 14 is preferably adapted for inversion of arms 53 so that free ends 54 point in a second direction, opposite to the first direction in which the free ends 54 pointed in the closed position, each arm 53 forming an obtuse angle relative to axis 21 as illustrated in FIG. 7. The arms 53 may be rotated so that the engagement surfaces 50 are disposed at a separation angle 56 of up to 360 degrees, and preferably at least up to 270 degrees. This may be accomplished by exerting a force against actuation mechanism 58 with a push rod or plunger extending through coupling member 19 as described above. In this embodiment, once the distal elements 18 have rotated beyond 180 degrees apart, the spring loading of the actuation mechanism 58 biases the distal elements 18 toward the inverted position. The spring loading of the actuation mechanism 58 resists outward movement of the actuation mechanism 58 and urges the device 14 towards the inverted position.

With arms 53 in the inverted position, engagement surfaces 50 provide an atraumatic surface deflect tissues as the fixation device is withdrawn. This allows the device to be retracted back through the valve annulus without risk of injury to valvular and other tissues. In some cases, once the fixation device 14 has been pulled back through the valve, it will be desirable to return the device to the closed position for withdrawal of the device from the body (either through the vasculature or through a surgical opening).

The embodiment illustrated in FIGS. 5, 6A-B and 7 is assembled from separate components composed of biocompatible materials. The components may be formed from the same or different materials, including but not limited to stainless steel or other metals, Elgiloy^{®}, nitinol, titanium, tantalum, metal alloys or polymers. Additionally, some or all of these components may be made of bioabsorbable materials that will be absorbed by surrounding tissues or will dissolve into the bloodstream following implantation. It has been found that in mitral valve repair applications the fixation devices of the disclosure are completely surrounded by tissue within a few months of implantation, after which the devices could dissolve or be absorbed without negative impact to the repair.

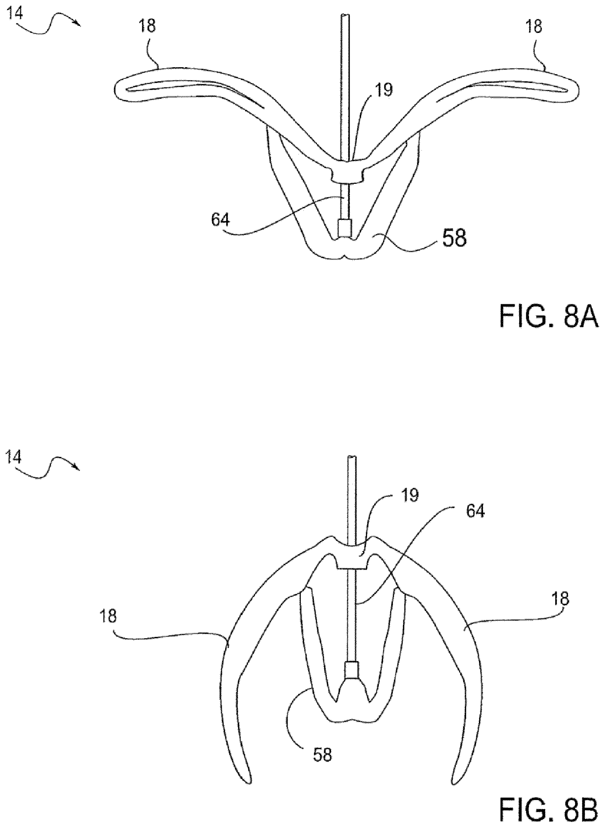

In a further embodiment, some or all of the components may be molded as one part, as illustrated in FIGS. 8A-8B. Here, the coupling member 19, distal elements 18 and actuation mechanism 58 of the fixation device 14 are all molded from a polymer material as one movable piece. FIG. 8A shows the fixation device 14 in the open position. Advancement of an actuator rod 64 rotates the distal elements 18 relative to the coupling member 19 by a living hinge or by elastic deformation of the plastic at the point of connection between the elements 18 and the coupling member 19. Typically, this point of connection comprises a thinner segment of polymer to facilitate such bending. Likewise, the actuation mechanism 58 coupled to the distal elements 18 in the same manner. FIG. 8B shows the fixation device 14 in the inverted position.

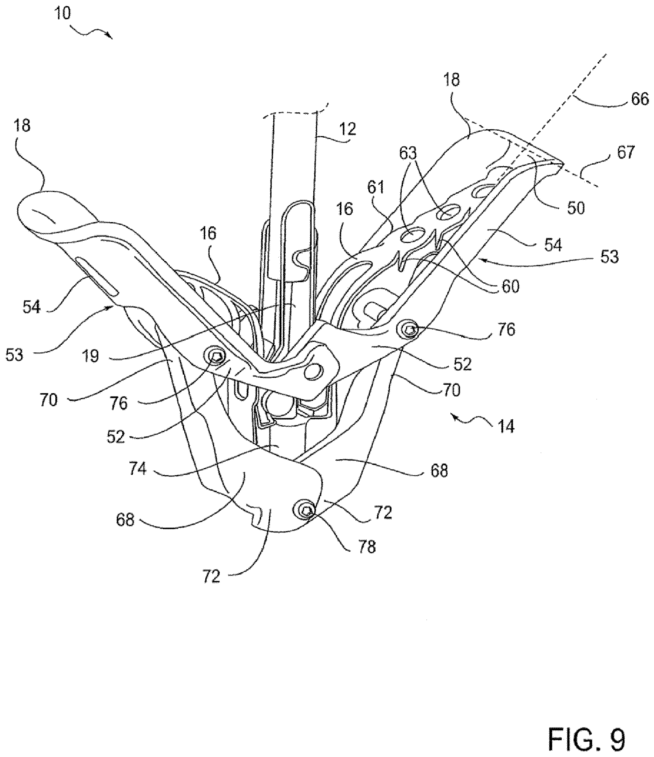

FIG. 9 illustrates another embodiment of a fixation device 14. Here, the fixation device 14 is shown coupled to a shaft 12 to form an interventional tool 10. The fixation device 14 includes a coupling member 19 and a pair of opposed distal elements 18. The distal elements 18 comprise elongate arms 53, each arm having a proximal end 52 rotatably connected to the coupling member 19 and a free end 54. The free ends 54 have a rounded shape to minimize interference with and trauma to surrounding tissue structures. Preferably, each free end 54 defines a curvature about two axes, one being an axis 66 perpendicular to longitudinal axis of arms 53. Thus, the engagement surfaces 50 have a cupped or concave shape to surface area in contact with tissue and to assist in grasping and holding the valve leaflets. This further allows arms 53 to nest around the shaft 12 in the closed position to minimize the profile of the device. Preferably, arms 53 are at least partially cupped or curved inwardly about their longitudinal axes 66. Also, preferably, each free end 54 defines a curvature about an axis 67 perpendicular to axis 66 or the longitudinal axis of arms 53. This curvature is a reverse curvature along the most distal portion of the free end 54. Likewise, the longitudinal edges of the free ends 54 may flare outwardly. Both the reverse curvature and flaring minimize trauma to the tissue engaged therewith.

In a preferred embodiment suitable for mitral valve repair, the transverse width across engagement surfaces 50 (which determines the width of tissue engaged) is at least about 2 mm, usually 3-10 mm, and preferably about 4-6 mm. In some situations, a wider engagement is desired wherein the engagement surfaces 50 are larger, for example about 2 cm, or multiple fixation devices are used adjacent to each other. Arms 53 and engagement surfaces 50 are configured to engage a length of tissue of about 4-10 mm, and preferably about 6-8 mm along the longitudinal axis of arms 53. Arms 53 further include a plurality of openings to enhance grip and to promote tissue ingrowth following implantation.

The valve leaflets are grasped between the distal elements 18 and proximal elements 16. In some embodiments, the proximal elements 16 are flexible, resilient, and cantilevered from coupling member 19. The proximal elements are preferably resiliently biased toward the distal elements. Each proximal element 16 is shaped and positioned to be at least partially recessed within the concavity of the distal element 18 when no tissue is present. When the fixation device 14 is in the open position, the proximal elements 16 are shaped such that each proximal element 16 is separated from the engagement surface 50 near the proximal end 52 of arm 53 and slopes toward the engagement surface 50 near the free end 54 with the free end of the proximal element contacting engagement surface 50, as illustrated in FIG. 9. This shape of the proximal elements 16 accommodates valve leaflets or other tissues of varying thicknesses.

Proximal elements 16 include a plurality of openings 63 and scalloped side edges 61 to increase grip on tissue. The proximal elements 16 optionally include frictional accessories, frictional features or grip-enhancing elements to assist in grasping and/or holding the leaflets. In preferred embodiments, the frictional accessories comprise barbs 60 having tapering pointed tips extending toward engagement surfaces 50. It may be appreciated that any suitable frictional accessories may be used, such as prongs, windings, bands, barbs, grooves, channels, bumps, surface roughening, sintering, high-friction pads, coverings, coatings or a combination of these.

Optionally, magnets may be present in the proximal and/or distal elements. It may be appreciated that the mating surfaces will be made from or will include material of opposite magnetic charge to cause attraction by magnetic force. For example, the proximal elements and distal elements may each include magnetic material of opposite charge so that tissue is held under constant compression between the proximal and distal elements to facilitate faster healing and ingrowth of tissue. Also, the magnetic force may be used to draw the proximal elements 16 toward the distal elements 18, in addition to or alternatively to biasing of the proximal elements toward the distal elements. This may assist in deployment of the proximal elements 16. In another example, the distal elements 18 each include magnetic material of opposite charge so that tissue positioned between the distal elements 18 is held therebetween by magnetic force.

The proximal elements 16 may be covered with a fabric or other flexible material as described below to enhance grip and tissue ingrowth following implantation. Preferably, when fabrics or coverings are used in combination with barbs or other frictional features, such features will protrude through such fabric or other covering so as to contact any tissue engaged by proximal elements 16.

In an exemplary embodiment, proximal elements 16 are formed from metallic sheet of a spring-like material using a stamping operation which creates openings 63, scalloped edges 61 and barbs 60. Alternatively, proximal elements 16 could be comprised of a spring-like material or molded from a biocompatible polymer. It should be noted that while some types of frictional accessories that can be used in the present disclosure may permanently alter or cause some trauma to the tissue engaged thereby, in a preferred embodiment, the frictional accessories will be atraumatic and will not injure or otherwise affect the tissue in a clinically significant way. For example, in the case of barbs 60, it has been demonstrated that following engagement of mitral valve leaflets by fixation device 14, should the device later be removed during the procedure barbs 60 leave no significant permanent scarring or other impairment of the leaflet tissue and are thus considered atraumatic.

The fixation device 14 also includes an actuation mechanism 58. In this embodiment, the actuation mechanism 58 comprises two link members or legs 68, each leg 68 having a first end 70 which is rotatably joined with one of the distal elements 18 at a riveted joint 76 and a second end 72 which is rotatably joined with a stud 74. The legs 68 are preferably comprised of a rigid or semi-rigid metal or polymer such as Elgiloy^{®}, cobalt chromium or stainless steel, however any suitable material may be used. While in the embodiment illustrated both legs 68 are pinned to stud 74 by a single rivet 78, it may be appreciated, however, that each leg 68 may be individually attached to the stud 74 by a separate rivet or pin. The stud 74 is joinable with an actuator rod 64 (not shown) which extends through the shaft 12 and is axially extendable and retractable to move the stud 74 and therefore the legs 68 which rotate the distal elements 18 between closed, open and inverted positions. Likewise, immobilization of the stud 74 holds the legs 68 in place and therefore holds the distal elements 18 in a desired position. The stud 74 may also be locked in place by a locking feature which will be further described in later sections.

In any of the embodiments of fixation device 14 disclosed herein, it may be desirable to provide some mobility or flexibility in distal elements 18 and/or proximal elements 16 in the closed position to enable these elements to move or flex with the opening or closing of the valve leaflets. This provides shock absorption and thereby reduces force on the leaflets and minimizes the possibility for tearing or other trauma to the leaflets. Such mobility or flexibility may be provided by using a flexible, resilient metal or polymer of appropriate thickness to construct the distal elements 18. Also, the locking mechanism of the fixation device (described below) may be constructed of flexible materials to allow some slight movement of the proximal and distal elements even when locked. Further, the distal elements 18 can be connected to the coupling mechanism 19 or to actuation mechanism 58 by a mechanism that biases the distal element into the closed position (inwardly) but permits the arms to open slightly in response to forces exerted by the leaflets. For example, rather than being pinned at a single point, these components may be pinned through a slot that allowed a small amount of translation of the pin in response to forces against the arms. A spring is used to bias the pinned component toward one end of the slot.

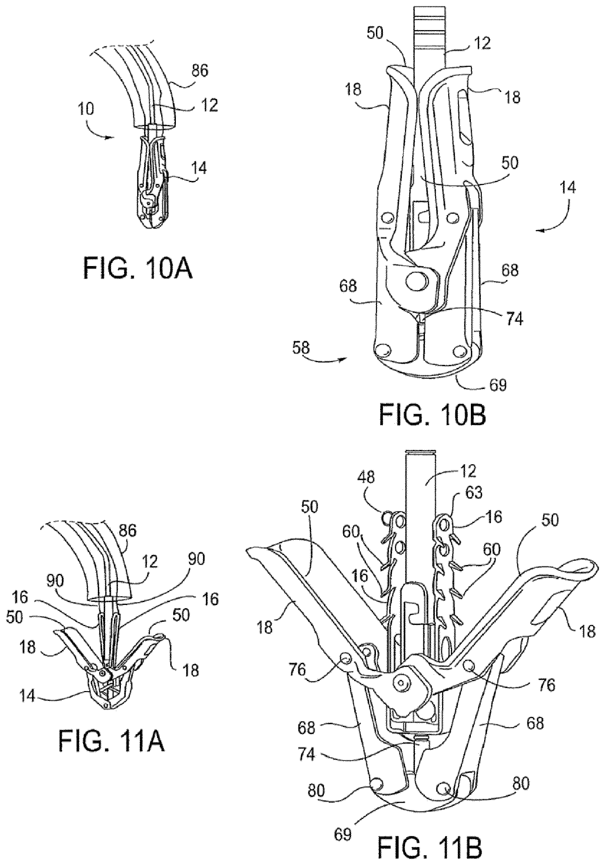

FIGS. 10A-10B, 11A-11B, 12A-12B, 13A-13B, and FIGS. 14-16 illustrate embodiments of the fixation device 14 of FIG. 9 in various possible positions during introduction and placement of the device 14 within the body to perform a therapeutic procedure. FIG. 10A illustrates an embodiment of an interventional tool 10 delivered through a catheter 86. It may be appreciated that the interventional tool 10 may take the form of a catheter, and likewise, the catheter 86 may take the form of a guide catheter or sheath. However, in this example the terms interventional tool 10 and catheter 86 will be used. The interventional tool 10 comprises a fixation device 14 coupled to a shaft 12 and the fixation device 14 is shown in the closed position. FIG. 10B illustrates a similar embodiment of the fixation device of FIG. 10A in a larger view. In the closed position, the opposed pair of distal elements 18 are positioned so that the engagement surfaces 50 face each other. Each distal element 18 comprises an elongate arm 53 having a cupped or concave shape so that together the arms 53 surround the shaft 12 and optionally contact each other on opposite sides of the shaft. This provides a low profile for the fixation device 14 which is readily passable through the catheter 86 and through any anatomical structures, such as the mitral valve. In addition, FIG. 10B further includes an actuation mechanism 58. In this embodiment, the actuation mechanism 58 comprises two legs 68 which are each movably coupled to a base 69. The base 69 is joined with an actuator rod 64 which extends through the shaft 12 and is used to manipulate the fixation device 14. In some embodiments, the actuator rod 64 attaches directly to the actuation mechanism 58, particularly the base 69. However, the actuator rod 64 may alternatively attach to a stud 74 which in turn is attached to the base 69. In some embodiments, the stud 74 is threaded so that the actuator rod 64 attaches to the stud 74 by a screw-type action. However, the rod 64 and stud 74 may be joined by any mechanism which is releasable to allow the fixation device 14 to be detached from shaft 12.

FIGS. 11A-11B illustrate the fixation device 14 in the open position. In the open position, the distal elements 18 are rotated so that the engagement surfaces 50 face a first direction. Distal advancement of the stud 74 relative to coupling member 19 by action of the actuator rod 64 applies force to the distal elements 18 which begin to rotate around joints 76 due to freedom of movement in this direction. Such rotation and movement of the distal elements 18 radially outward causes rotation of the legs 68 about joints 80 so that the legs 68 are directly slightly outwards. The stud 74 may be advanced to any desired distance correlating to a desired separation of the distal elements 18. In the open position, engagement surfaces 50 are disposed at an acute angle relative to shaft 12, and are preferably at an angle of between 90 and 180 degrees relative to each other. In one embodiment, in the open position the free ends 54 of arms 53 have a span therebetween of about 10-20 mm, usually about 12-18 mm, and preferably about 14-16 mm.

Proximal elements 16 are typically biased outwardly toward arms 53. The proximal elements 16 may be moved inwardly toward the shaft 12 and held against the shaft 12 with the aid of proximal element lines 90 which can be in the form of sutures, wires, nitinol wire, rods, cables, polymeric lines, or other suitable structures. The proximal element lines 90 may be connected with the proximal elements 16 by threading the lines 90 in a variety of ways. When the proximal elements 16 have a loop shape, as shown in FIG. 11A, the line 90 may pass through the loop and double back. When the proximal elements 16 have an elongate solid shape, as shown in FIG. 11B, the line 90 may pass through one or more of the openings 63 in the element 16. Further, a line loop 48 may be present on a proximal element 16, also illustrated in FIG. 11B, through which a proximal element line 90 may pass and double back. Such a line loop 48 may be useful to reduce friction on proximal element line 90 or when the proximal elements 16 are solid or devoid of other loops or openings through which the proximal element lines 90 may attach. A proximal element line 90 may attach to the proximal elements 16 by detachable means which would allow a single line 90 to be attached to a proximal element 16 without doubling back and would allow the single line 90 to be detached directly from the proximal element 16 when desired. Examples of such detachable means include hooks, snares, clips or breakable couplings, to name a few. By applying sufficient tension to the proximal element line 90, the detachable means may be detached from the proximal element 16 such as by breakage of the coupling. Other mechanisms for detachment may also be used. Similarly, a lock line 92 may be attached and detached from a locking mechanism by similar detachable means.

In the open position, the fixation device 14 can engage the tissue which is to be approximated or treated. The embodiment illustrated in FIGS. 9-11 is adapted for repair of the mitral valve using an antegrade approach from the left atrium. The interventional tool 10 is advanced through the mitral valve from the left atrium to the left ventricle. The distal elements 18 are oriented to be perpendicular to the line of coaptation and then positioned so that the engagement surfaces 50 contact the ventricular surface of the valve leaflets, thereby grasping the leaflets. The proximal elements 16 remain on the atrial side of the valve leaflets so that the leaflets lie between the proximal and distal elements. In this embodiment, the proximal elements 16 have frictional accessories, such as barbs 60 which are directed toward the distal elements 18. However, neither the proximal elements 16 nor the barbs 60 contact the leaflets at this time.

The interventional tool 10 may be repeatedly manipulated to reposition the fixation device 14 so that the leaflets are properly contacted or grasped at a desired location. Repositioning is achieved with the fixation device in the open position. In some instances, regurgitation may also be checked while the device 14 is in the open position. If regurgitation is not satisfactorily reduced, the device may be repositioned and regurgitation checked again until the desired results are achieved.

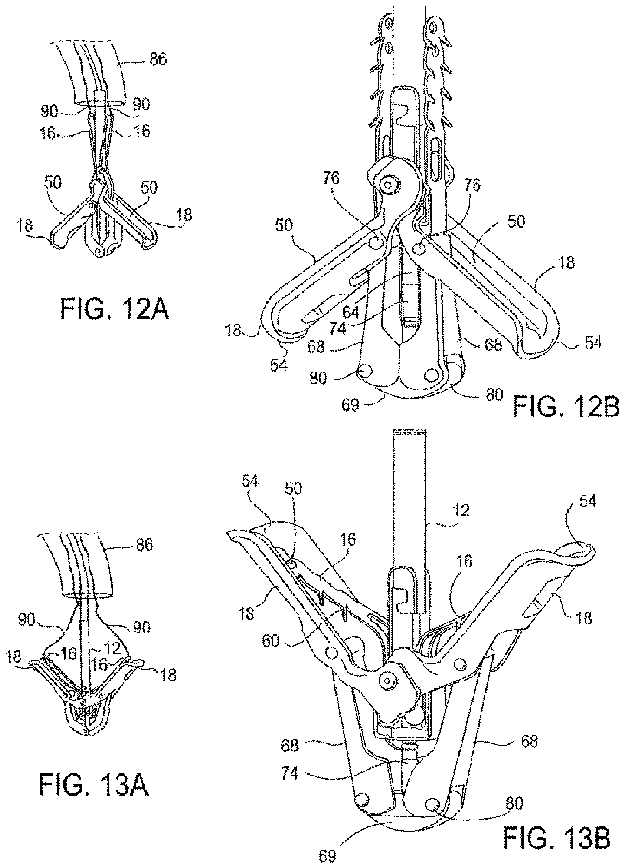

It may also be desired to invert the fixation device 14 to aid in repositioning or removal of the fixation device 14. FIGS. 12A-12B illustrate the fixation device 14 in the inverted position. By further advancement of stud 74 relative to coupling member 19, the distal elements 18 are further rotated so that the engagement surfaces 50 face outwardly and free ends 54 point distally, with each arm 53 forming an obtuse angle relative to shaft 12. The angle between arms 53 is preferably in the range of about 270 to 360 degrees. Further advancement of the stud 74 further rotates the distal elements 18 around joints 76. This rotation and movement of the distal elements 18 radially outward causes rotation of the legs 68 about joints 80 so that the legs 68 are returned toward their initial position, generally parallel to each other. The stud 74 may be advanced to any desired distance correlating to a desired inversion of the distal elements 18. Preferably, in the fully inverted position, the span between free ends 54 is no more than about 20 mm, usually less than about 16 mm, and preferably about 12-14 mm. In this illustration, the proximal elements 16 remain positioned against the shaft 12 by exerting tension on the proximal element lines 90. Thus, a relatively large space may be created between the elements 16, 18 for repositioning. In addition, the inverted position allows withdrawal of the fixation device 14 through the valve while minimizing trauma to the leaflets. Engagement surfaces 50 provide an atraumatic surface for deflecting tissue as the fixation device is refracted proximally. It should be further noted that barbs 60 are angled slightly in the distal direction (away from the free ends of the proximal elements 16), reducing the risk that the barbs will catch on or lacerate tissue as the fixation device is withdrawn.

Once the fixation device 14 has been positioned in a desired location against the valve leaflets, the leaflets may then be captured between the proximal elements 16 and the distal elements 18. FIGS. 13A-13B illustrate the fixation device 14 in such a position. Here, the proximal elements 16 are lowered toward the engagement surfaces 50 so that the leaflets are held therebetween. In FIG. 13B, the proximal elements 16 are shown to include barbs 60 which may be used to provide atraumatic gripping of the leaflets. Alternatively, larger, more sharply pointed barbs or other penetration structures may be used to pierce the leaflets to more actively assist in holding them in place. This position is similar to the open position of FIGS. 11A-11B, however the proximal elements 16 are now lowered toward arms 53 by releasing tension on proximal element lines 90 to compress the leaflet tissue therebetween. At any time, the proximal elements 16 may be raised and the distal elements 18 adjusted or inverted to reposition the fixation device 14, if regurgitation is not sufficiently reduced.

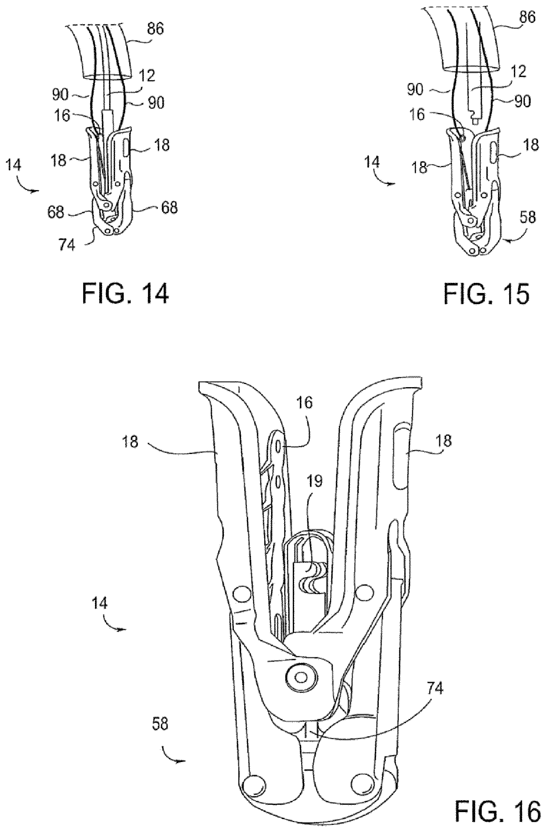

After the leaflets have been captured between the proximal and distal elements 16, 18 in a desired arrangement, the distal elements 18 may be locked to hold the leaflets in this position or the fixation device 14 may be returned to or toward a closed position. Such locking will be described in a later section. FIG. 14 illustrates the fixation device 14 in the closed position wherein the leaflets (not shown) are captured and coapted. This is achieved by retraction of the stud 74 proximally relative to coupling member 19 so that the legs 68 of the actuation mechanism 58 apply an upwards force to the distal elements 18 which in turn rotate the distal elements 18 so that the engagement surfaces 50 again face one another. The released proximal elements 16 which are biased outwardly toward distal elements 18 are concurrently urged inwardly by the distal elements 18. The fixation device 14 may then be locked to hold the leaflets in this closed position as described below.

As shown in FIG. 15, the fixation device 14 may then be released from the shaft 12. As mentioned, the fixation device 14 is releasably coupleable to the shaft 12 by coupling member 19. FIG. 15 illustrates the coupling structure, a portion of the shaft 12 to which the coupling member 19 of the fixation device 14 attaches. As shown, the proximal element lines 90 may remain attached to the proximal elements 16 following detachment from shaft 12 to function as a tether to keep the fixation device 14 connected with the catheter 86. Optionally, a separate tether coupled between shaft 12 and fixation device 14 may be used expressly for this purpose while the proximal element lines 90 are removed. In any case, the repair of the leaflets or tissue may be observed by non-invasive visualization techniques, such as echocardiography, to ensure the desired outcome. If the repair is not desired, the fixation device 14 may be retrieved with the use of the tether or proximal element lines 90 so as to reconnect coupling member 19 with shaft 12.

In an exemplary embodiment, proximal element lines 90 are elongated flexible threads, wire, cable, sutures or lines extending through shaft 12, looped through proximal elements 16, and extending back through shaft 12 to its proximal end. When detachment is desired, one end of each line may be released at the proximal end of the shaft 12 and the other end pulled to draw the free end of the line distally through shaft 12 and through proximal element 16 thereby releasing the fixation device.

FIG. 16 illustrates a released fixation device 14 in a closed position. As shown, the coupling member 19 remains separated from the shaft 12 of the interventional tool 10 and the proximal elements 16 are deployed so that tissue (not shown) may reside between the proximal elements 16 and distal elements 18.

While the above-described embodiments of the disclosure utilize a push-to-open, pull-to-close mechanism for opening and closing distal elements 18, it should be understood that a pull-to-open, push-to-close mechanism is equally possible. For example, distal elements 18 may be coupled at their proximal ends to stud 74 rather than to coupling member 19, and legs 68 may be coupled at their proximal ends to coupling member 19 rather than to stud 74. In this example, when stud 74 is pushed distally relative to coupling member 19, distal elements 18 would close, while pulling on stud 74 proximally toward coupling member 19 would open distal elements 18.

### B. Covering on Fixation Device

The fixation device 14 may optionally include a covering. The covering may assist in grasping the tissue and may later provide a surface for tissue ingrowth. Ingrowth of the surrounding tissues, such as the valve leaflets, provides stability to the device 14 as it is further anchored in place and may cover the device with native tissue thus reducing the possibility of immunologic reactions. The covering may be comprised of any biocompatible material, such as polyethylene terephthalate, polyester, cotton, polyurethane, expanded polytetrafluoroethylene (ePTFE), silicon, or various polymers or fibers and have any suitable form, such as a fabric, mesh, textured weave, felt, looped or porous structure. Generally, the covering has a low profile so as not to interfere with delivery through an introducer sheath or with grasping and coapting of leaflets or tissue.

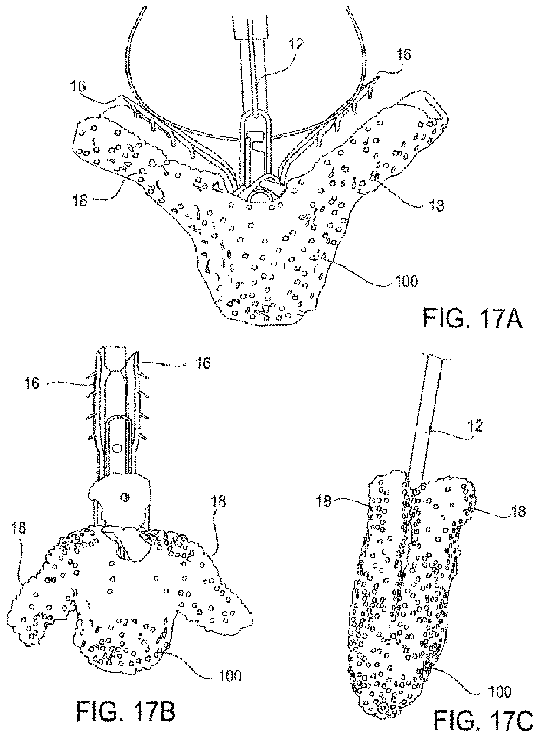

FIGS. 17A-17C illustrate a covering 100 on the fixation device 14 wherein the device 14 is in various positions. FIG. 17A shows the covering 100 encapsulating the distal elements 18 and the actuation mechanism 58 while the device 14 is in the open position. Thus, the engagement surfaces 50 are covered by the covering 100 which helps to minimize trauma on tissues and provides additional friction to assist in grasping and retaining tissues. FIG. 17B shows the device 14 of FIG. 17A in the inverted position. The covering 100 is loosely fitted and/or is flexible or elastic such that the device 14 can freely move to various positions and the covering 100 conforms to the contours of the device 14 and remains securely attached in all positions. FIG. 17C shows the device 14 in the closed position. Thus, when the fixation device 14 is left behind as an implant in the closed position, the exposed surfaces of the device 14 are substantially covered by the covering 100. It may be appreciated that the covering 100 may cover specific parts of the fixation device 14 while leaving other parts exposed. For example, the covering 100 may comprise sleeves that fit over the distal elements 18 and not the actuation mechanism 58, caps that fit over the distal ends 54 of the distal elements 18 or pads that cover the engagement surfaces 50, to name a few. It may be appreciated that, the covering 100 may allow any frictional accessories, such as barbs, to be exposed. Also, the covering 100 may cover the proximal elements 16 and/or any other surfaces of the fixation device 14. In any case, the covering 100 should be durable to withstand multiple introduction cycles and, when implanted within a heart, a lifetime of cardiac cycles.

The covering 100 may alternatively be comprised of a polymer or other suitable materials dipped, sprayed, coated or otherwise adhered to the surfaces of the fixation device 14. Optionally, the polymer coating may include pores or contours to assist in grasping the tissue and/or to promote tissue ingrowth.

Any of the coverings 100 may optionally include drugs, antibiotics, anti-thrombosis agents, or anti-platelet agents such as heparin, COUMADINO (Warfarin Sodium), to name a few. These agents may, for example, be impregnated in or coated on the coverings 100. These agents may then be delivered to the grasped tissues surrounding tissues and/or bloodstream for therapeutic effects.

### C. Confirmation via Integrated Sensors

The disclosure above describes several variations of fixation devices and corresponding delivery devices for implanting the fixation devices within the native anatomy. As noted, edge-to-edge repair procedures are difficult, and the operator must typically manipulate multiple instruments while monitoring the leaflet insertion via an imaging modality. Due to difficulty of imaging highly mobile leaflets and imaging artifacts it is difficult to ensure adequate leaflet insertion and grasp. This may lead to higher probability of single leaflet device attachment (SLDA), or similar complications.

To address this concern, any of the fixation devices described above may optionally include one or more embedded sensors that can offer adequate verification of leaflet insertion and grasp, which may lead to more confident, safer, efficient and effective procedures. Generally, devices and methods to mitigate risk of SLDA due to incomplete or inadequate leaflet insertion or grasp are described. Current techniques are limited to transesophageal (TEE) imaging and the like to ensure a leaflet is adequately inserted and grasped during fixation device actuation. The use of embedded sensor(s) on the fixation device can offer additional mitigation in patients where leaflet visualization is challenging due to anatomical and procedural limitations.

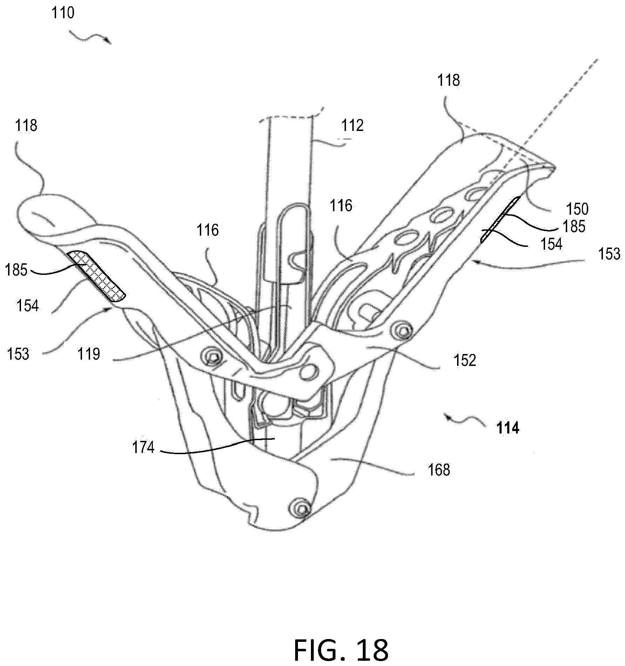

FIG. 18 illustrates one embodiment of a fixation device 114 having such sensors. By way of illustration, fixation device 114 may include structures and/or functions similar to those depicted in FIG. 9, but it will be understood that any of the variations previously described may be embedded with sensors. In addition, while FIGS. 9-17 depict exemplary tissue securement devices, similarly functioning devices that grasp and capture tissue with fixation elements may be embedded with sensors. In FIG. 18, previously-described elements of FIG. 9 have corresponding reference numeral preceding with a "1" (e.g., fixation device 114 is similar to fixation device 14). Fixation device 114 is shown coupled to a shaft 112 to form an interventional tool 110. The fixation device 114 includes a coupling member 119 and a pair of opposed distal elements 118. The distal elements 118 comprise elongate arms 153, each arm having a proximal end 152 rotatably operatively connected to the coupling member 119 via stud 174 and a free end 154. The free ends 154 have an engagement surface 150, and a rounded shape to minimize interference with and trauma to surrounding tissue structures. The valve leaflets may be grasped between the distal elements 118 and proximal elements 116. In some embodiments, the proximal elements 116 are flexible, resilient, and cantilevered from coupling member 119. The proximal elements are preferably resiliently biased toward the distal elements. In this example, fixation device 114 also includes an actuation mechanism including legs 168 as previously described.

In this embodiment, an embedded sensor 185 may be disposed on one or both of the elongate arms 153. In some examples, each arm 153 may include one or more (two, three, four of five) sensors along its length or width to increase sensing resolution. In some examples, sensors 185 include one or more microelectromechanical system (MEMS) based sensors or similar, that can transduce a detectable signal due to physical contact with tissue (e.g., leaflet), change in any one or more of electrical, optical, resistive, and impedance properties, change in pressure, change in optical properties of surrounding tissue or environment, change in acoustic properties of surrounding tissue or environment, or change in mechanical forces on components due to contact with tissue. Such sensors may be embedded, integrated, or coupled to any component of the fixation device.

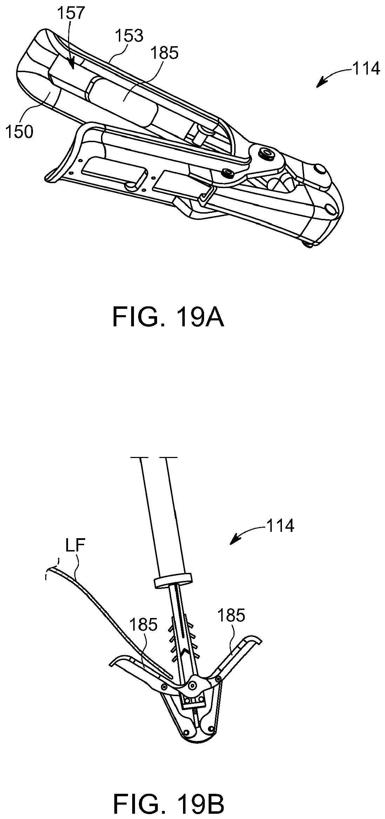

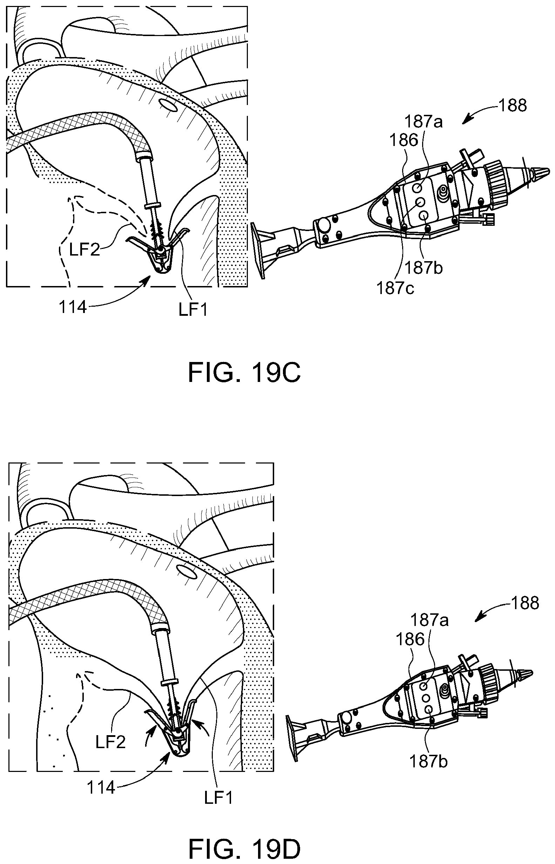

In one variation, shown in FIGS. 19A-B, fixation device 114 includes a pair of arms, and each arm 153 defines an elongated window 157 extending along the arm's longitudinal axis, the sensors 185 being disposed within the windows. In this example, sensor 185 is integrated with the arm and specifically, within or adjacent to, or defines the arm's engagement surface 150 such that a leaflet LF that contacts the sensor will create a detectable signal. As previously described a number of different sensor(s) and one or more properties of the sensor and/or tissue may be utilized to generate a signal that can be detected and processed to interpret appropriate tissue contact and/or grasp of one or more leaflets. In some examples, the signal detection may be through a wired system that extends through shaft 112 and is decoupleable therewith, or through wireless means.

The detected signal may be collected, interpreted and/or displayed as numerical values, plots and/or simple visual indicators during the procedure. For example, the data may include a time series and/or discreet events collected, recorded, interpreted and/or displayed. In some examples, the system may include relaying this information or data on a graphical user interface in real-time to observe relevant information, such as contact with the leaflets. Turning to FIG. 19C, in at least some examples, indicators may be disposed on a handle 188 of the delivery device, and the indicators may communicate a location, property or status of the fixation device 114 based on its distance and/or contact with a leaflet LF. For example, handle 188 may include an indicator 186 in the form of one or more lights 187a,187b (e.g., LEDs) that will signal when the leaflet is in contact with sensor 185 on fixation device 114.

As seen in FIG. 19C, indicator lights 187a,187b each correspond to a particular arm of fixation device 114. Indicator light 187a is shown as displaying a green light to signal that a leaflet LF1 is in contact with, or sufficiently close to, a sensor of the arm, and indicator light 187b is shown as displaying a red light to signal that a leaflet LF2 is not in contact with, or not close enough to, the particular corresponding sensor. The user may manipulate the device to achieve better position of the fixation device 114 with respect to one or both leaflets. When the leaflet LF2 does come in contact with the sensor, the light 187b may turn green, and the user may begin the process of deploying the fixation device 114 knowing that both leaflets LF1,LF2 will be properly captured therein. Additionally, an optional middle light 187c may be used to indicate that the system is ready (or reset for grasping in case of multiple grasping events) for the detection of a leaflet. The middle light 187c may also signal that the clip arm angles are at the right position to initiate grasping to serve as "ready to grasp" indicator.

In some examples, the signals and/or collected data generated by the embedded sensors may be analyzed by a computer having a storage and a processor, utilizing software algorithms including artificial intelligence algorithms to provide guidance for successful leaflet insertion to the user. For example, based on the collected data, one or more preferred approaches for leaflet insertion optimization, clip placement or coaptation area may be calculated. The data analysis may also include correlation of sensor signal response with imaging, EKG and/or other time series information to ensure leaflet contact with device is synchronized with the heart motion or visual information available for implanters. Additionally, the sensor signal and/or data may be presented in a complementary manner to the imaging information (e.g., by being super positioned on imaging data) to help an operator position, steer, navigate and/or interact with anatomy. This time series or discreet information may be integrated on the same display or separate displays. Additionally, the signals and/or data may be converted into a visual, haptic or audible feedback (e.g., an audible chime or click from the handle) to the user to help them navigate and deploy fixation device 114. In addition, sensor feedback can be monitored over time to communicate dynamic information to inform the implanter as to whether tissue contact is steady over time or fluctuating during the cardiac cycle as biomechanical loads or pressures are applied to a device-tissue interface. In this case, if a sensor indicates contact for a sufficient time threshold (80%-100% of a time interval such as a cardiac cycle), feedback can be provided to the user to inform them that the tissue contact is steady and reliable. In one example, steady tissue contact could be communicated as a solid light or display readout, as compared to a flashing light or display readout. In another example, a favorable color indicator may be presented if steady contact is determined by the sensor(s). In cases where tissue contact is sensed for less than 50% of a cardiac cycle (or other relevant time interval, such as multiple cardiac cycles), a less favorable color indicator could be presented, such as a blinking or red light.

### D. Confirmation via Mechanical Indicator

As described above, during a valve repair procedures predictable, consistent and observable grasp of valve leaflet is desirable for better outcomes and procedure effectiveness. The system above describes integrated sensors for guidance and/or confirmation to ensure proper leaflet positioning within a fixation device. In another variation, a mechanical indicator may be used. In this embodiment, an embedded mechanical indicator may offer verification of a leaflet coming in contact with components visible in ultrasound or in x-ray-based modality to provide additional assurance of leaflet insertion and grasp. This may result in more confident, safer, efficient and effective procedures. The use of a mechanical indicator visible with ultrasound or X-ray or both could provide additional mitigation in patients where leaflet visualization is challenging due to anatomical and procedural limitations.

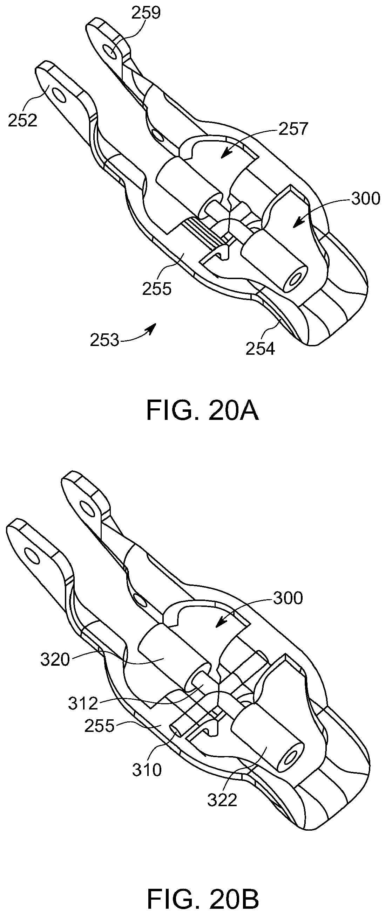

FIGS. 20A-B illustrates another embodiment of an elongated arm 253 of a fixation device. The remainder of fixation device may include structures and/or functions as previously described. As shown in FIG. 20A, elongated arm 253 may extend between a proximal end 252 rotatably connectable to a coupling member (not shown) via an aperture 259 and a free end 254. In this embodiment, each arm 253 defines an elongated window 257 extending along the arm's longitudinal axis and a transverse bridge 255 that extends orthogonal to the longitudinal axis of the arm and spans ends of the arm. A mechanical indicator 300 is coupled to elongated arm 253 at transverse bridge 255. As best shown in FIG. 20B, indicator 300 may include a crossbrace 310 that couples to ends of bridge 255. In some examples, portions of crossbrace 310 pass through opposing orifices 265 of bridge 255 to couple the two elements together. Orifices 265 may be large enough to allow rotation of crossbrace 310 with respect thereto. Crossbrace 310 may be connected or unitarily formed with axial beam 312 so that the two members are orthogonal relative to one another and form a cruciform shape. A first marker 320 and a second marker 322 may be coupled to opposing ends of beam 312. Markers 320,322 may be glued, tubing welded or swaged onto beam 312. In some examples, the two markers 320,322 are of a similar or same shape and/or size to each other, or they may be distinct. The two markers are color-coded here by way of illustration. In some examples, first marker 320 is a spacer and second marker 322 is radiopaque or echogenic, or vice versa. Alternatively, both first and second markers 320,322 are radiopaque or echogenic. The radiopaque markers may comprise ELGILOY @ alloy, platinum iridium, tantalum or any other suitable material.

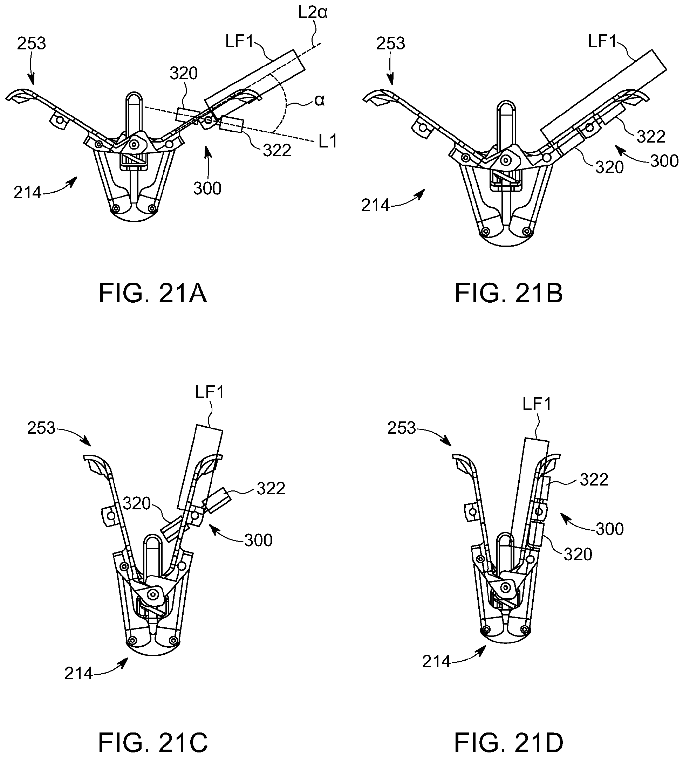

Mechanical indicator 300 may be designed to be visible to reflect adequate leaflet insertion within a fixation device. In some examples, the indicator 300 may become visible during the insertion of the leaflet and then rotate to a non-visible configuration indicating that leaflet is properly inserted, adding to the security of leaflet insertion step. FIGS. 21A-D illustrate the position of indicator 300 at various possible positions during introduction and placement of the device within the body to perform a therapeutic procedure. FIGS. 21A-B show fixation device 214 in the open condition, and a leaflet LF1 is shown sliding over elongated arm 253. As shown in FIG. 21A, indicator 300 is not aligned with elongated arm 253 without the leaflet in place. That is, the longitudinal axis L1 of indicator 300 and the longitudinal axis L2 of elongated arm 253 form an indicator angle α of approximately 45 degrees. This difference in position or angular offset between indicator 300 and elongated arm 253 may be visible during the procedure with ultrasound or X-ray. In some examples, an arm 253 may appear to have a continuous edge during imaging when indicator 300 is properly aligned therewith during the presence of a leaflet, and will appear to have a discontinuous edge when proper insertion has not been achieved. In some examples, indicator 300 is biased to an indicator angle of greater than 5 degrees absent an external force (e.g., when no leaflet is present). Turning to FIG. 21B, as leaflet LF1 slides in place over elongated arm 253, it pushes first marker 320 down and urges indicator 300 to rotate and align with elongated arm 253 to take a non-visible position. In some examples, when the leaflet LF1 is properly aligned within fixation device 214, the angle between the indicator and the elongated arm is between 0 and 15 degrees. In some examples, an angle of less than 5 degrees may serve to confirm that the leaflet in proper position.

As shown in FIGS. 21C-D, once the leaflet LF1 is fully inserted (e.g., it is inserted distally beyond crossbrace 310), the indicator 300 may continue to provide information about its position. For example, in FIG. 21C, the leaflet LF1 has slipped out of fixation device 214 and is improperly positioned over elongated arm 253 and indicator 300 has been misaligned with the corresponding arm. The operator may need to reposition the device or the leaflet until the leaflet LF1 lays flat over elongated arm 253, which will be clear from the position of indicator 300 being aligned with the arm (FIG. 21D).

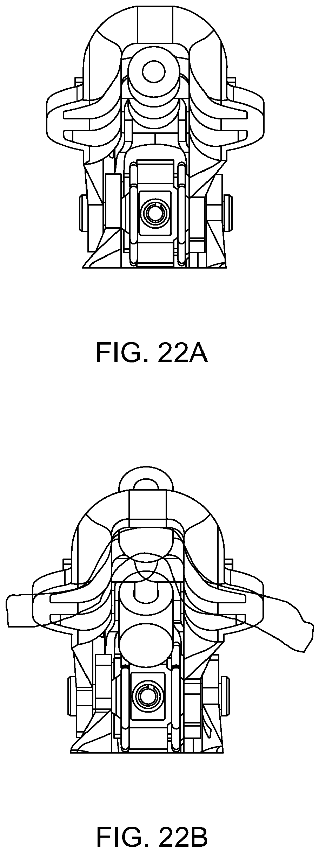

Variations of this concept are possible. For examples, a mechanical rotation hard stop may be used to limit the angle of rotation of indicator 300 with respect to elongated arm 253. For example, a clip cover can be used to limit rotation of leaflet insertion indicator to +/- 45 degrees with respect to the longitudinal axis L2 of elongated arm 253. Additionally, it will be understood that although a single indicator 300 is shown, each arm 253 may have a dedicated indicator to indicate the position of a corresponding leaflet with respect to that arm, and that both indicators on the fixation device may be monitored in real-time. Additionally, fixation sensors may provide information about the inserted leaflet intraprocedurally and at timeframes long after device implantation. At longer timeframes, fixation sensors can provide diagnostic or predictive information regarding the device-tissue interface (and possible failure) and prompt a medical practitioner in judging if an additional intervention should be planned to restore tissue attachment to the repair device. FIGS. 22A-B show a short axis view of the fixation device without the leaflet (FIG. 22A), and where the sensor is pushed outside of the body of the fixation implant when leaflets are adequately positioned inside it (FIG. 22B).

It is to be understood that the embodiments described herein are merely illustrative of the principles and applications of the present disclosure. For example, a system may include both mechanical and electromechanical or capacitive sensing indicating elements. Additionally, a system may include both mechanical and electrical sensing elements. Moreover, certain components are optional, and the disclosure contemplates various configurations and combinations of the elements disclosed herein. It is therefore to be understood that numerous modifications may be made to the illustrative embodiments and that other arrangements may be devised without departing from the spirit and scope of the present disclosure as defined by the appended claims.

Although the disclosure herein has been described with reference to particular embodiments, it is to be understood that these embodiments are merely illustrative of the principles and applications of the present disclosure. It is therefore to be understood that numerous modifications may be made to the illustrative embodiments and that other arrangements may be devised without departing from the spirit and scope of the present disclosure as defined by the appended claims.

Various examples are set out in the following numbered paragraphs (NPs):
NP1. A valve clip, comprising: a stud; two arms operatively coupled to the stud and capable of transitioning between an open condition and a closed condition; and at least one sensor coupled to each of the two arms.
NP2. The valve clip of NP1, wherein each of the at least one sensor includes a microelectromechanical sensor.
NP3. The valve clip of NP1 or NP2, wherein the at least one sensor is capable of detecting contact with tissue.
NP4. The valve clip of any of NP1 to NP3, wherein the at least one sensor is capable of detecting a change in at least one of an electrical, resistive, and impedance property.
NP5. The valve clip of any of NP1 to NP4, wherein the at least one sensor is capable of detecting a change in at least one of a pressure, an optical property of surrounding tissue, an acoustic property of surrounding tissue, or a mechanical force on a component of the valve clip.
NP6. The valve clip of any of NP1 to NP5, wherein each of the two arms defines a window, and the at least one sensor is disposed within a corresponding window.
NP7. A system comprising: the valve clip of NP1; and a handle of a delivery device having an indicator to relay information from the at least one sensor.
NP8. The system of NP7, wherein the indicator comprises two lights to signal appropriate leaflet positioning adjacent to each of the two arms.
NP9. The system of NP7 or NP8, wherein the indicator comprises an audible alert to signal appropriate leaflet positioning adjacent to each of the two arms.
NP10. A valve clip, comprising: a stud; two arms operatively coupled to the stud and capable of transitioning between an open condition and a closed condition; an indicator coupled to each of the two arms and moveable relative thereto.
NP11. The valve clip of NP10, wherein each of the two arms defines a window and a bridge, the indicator being coupled to the bridge of a corresponding arm.
NP12. The valve clip of NP10 or NP11, wherein each indicator is rotatable relative to a corresponding arm.
NP13. The valve clip of any of NP10 to NP12, wherein each indicator includes a crossbrace, a beam and two markers coupled to opposing ends of the beam.
NP14. The valve clip of any of NP10 to NP13, wherein each indicator has a first longitudinal axis, and a corresponding arm has a second longitudinal axis, the first longitudinal axis and the second longitudinal axis defining an indicator angle.
NP15. The valve clip of NP14, wherein the indicator angle is less than 5 degrees when a leaflet is properly disposed over the corresponding arm.
NP16. The valve clip of NP14 or NP15, wherein the indicator angle is greater than 5 degrees when no leaflet is present.
NP17. The valve clip of any of NP14 to NP16, wherein the indicator is biased to an indicator angle of greater than 5 degrees absent an external force.
NP18. The valve clip of any of NP10 to NP17, wherein the indicator is aligned with a corresponding arm when a leaflet is properly placed within the valve clip.
NP19. The valve clip of any of NP10 to NP18, wherein a portion of the indicator is radiopaque or echogenic.
NP20. The valve clip of NP13 and optionally also any of NP14 to NP19, wherein at least one of the two markers is radiopaque or echogenic.

## Claims

1. A valve clip, comprising:
a stud;
two arms operatively coupled to the stud and capable of transitioning between an open condition and a closed condition; and
at least one sensor coupled to each of the two arms.

2. The valve clip of claim 1, wherein each of the at least one sensor includes a microelectromechanical sensor.

3. The valve clip of claim 1 or 2, wherein the at least one sensor is capable of detecting contact with tissue.

4. The valve clip of any of the preceding claims, wherein the at least one sensor is capable of detecting a change in at least one of an electrical, resistive, and impedance property.

5. The valve clip of any of the preceding claims, wherein the at least one sensor is capable of detecting a change in at least one of a pressure, an optical property of surrounding tissue, an acoustic property of surrounding tissue, or a mechanical force on a component of the valve clip.

6. The valve clip of any of the preceding claims, wherein each of the two arms defines a window, and the at least one sensor is disposed within a corresponding window.

7. A system comprising:
the valve clip of claim 1; and
a handle of a delivery device having an indicator to relay information from the at least one sensor.

8. The system of claim 7, wherein the indicator comprises two lights to signal appropriate leaflet positioning adjacent to each of the two arms.

9. The system of claim 7 or 8, wherein the indicator comprises an audible alert to signal appropriate leaflet positioning adjacent to each of the two arms.
